# EUROPEAN PATENT SPECIFICATION

(11) **EP 3 157 590 B1**
(45) Date of publication and mention of the grant of the patent: **15.04.2020**
(21) Application number: 15741332.9
(22) Date of filing: 18.06.2015
(51) Int. Cl.: A61L 27/12, A61L 27/42, A61L 24/00, A61L 24/02

(54) **INJECTABLE APATITIC CEMENT IONICALLY MULTI-SUBSTITUTED FOR REGENERATIVE VERTEBROPLASTY AND KYPHOPLASTY**
INJIZIERBARER, ZUR REGENERATIVEN VERTEBROPLASTIE UND KYPHOPLASTIE IONISCH MEHRFACHSUBSTITUIERTER APATIT-ZEMENT
CIMENT APATITIQUE INJECTABLE IONIQUEMENT MULTI-SUBSTITUÉ POUR VERTÉBROPLASTIE ET CYPHOPLASTIE RÉGÉNÉRATIVES

(30) Priority: 19.06.2014 IT RM20140326
(43) Date of publication of application: 26.04.2017
(73) Proprietor: Consiglio Nazionale Delle Ricerche (C.N.R.), 00185 Roma (IT); Universita' Cattolica Del Sacro Cuore, 20123 Milano Mi (IT)
(72) Inventor: SPRIO, Simone, I-00185 Roma (IT); TAMPIERI, Anna, I-00185 Roma (IT); SANDRI, Monica, I-00185 Roma (IT); PANSERI, Silvia, I-00185 Roma (IT); LOGROSCINO, Giandomenico, I-20123 Milano (IT)
(74) Representative: Ghirardi, Valeria
(86) International application number: PCT/IB2015/054594
(87) International publication number: WO 2015/193836

(56) References cited:
- CN-A- 1 799 643
- US-A1- 2008 260 714
- AMIT BANDYOPADHYAY ET AL: "ZnO, SiO 2 , and SrO doping in resorbable tricalcium phosphates: Influence on strength degradation, mechanical properties, and in vitro bone-cell material interactions", JOURNAL OF BIOMEDICAL MATERIALS RESEARCH PART B: APPLIED BIOMATERIALS, vol. 100B, no. 8, 21 September 2012 (2012-09-21), pages 2203-2212, XP055168998, ISSN: 1552-4973, DOI: 10.1002/jbm.b.32789
- REID J W ET AL: "Synthesis and characterization of single-phase silicon-substituted alpha-tricalcium phosphate", BIOMATERIALS, ELSEVIER SCIENCE PUBLISHERS BV., BARKING, GB, vol. 27, no. 15, 1 May 2006 (2006-05-01), pages 2916-2925, XP027951023, ISSN: 0142-9612 [retrieved on 2006-05-01]

## Description

### TECHNICAL FIELD

The present invention relates to the field of bone regeneration therapies, particularly for use in bone regions, particularly spinal regions, characterized by bone loss or weakening as a result of injury, pharmacological treatment, tumours or osteoporosis (1, 2).

### BACKGROUND OF THE INVENTION

In cases of spinal lesions, such as fracture or collapse of vertebral bodies, vertebroplasty and kyphoplasty are established clinical procedures in which a malleable paste is injected into the bone defect and solidifies in a few minutes, thus providing physical and mechanical stabilization of the defect and allowing an early return to an erect posture. Thus it is possible to prevent the onset of secondary disturbances and pathologies caused by the immobility due to prolonged recovery, especially in older patients and/or those with reduced mobility.

However, the development of a cement suitable for vertebroplasty and kyphoplasty gives rise to considerable technological difficulties in relation to its use in clinical practice. The most significant difficulties relate to:
- injectability and cohesion: the injectable cement has to be capable of flowing through a cannula and appropriately filling the tortuous space of a cavity; moreover, it must not separate into solid and liquid fractions during injection; and
- setting times: the cement must be capable of hardening within appropriate periods for clinical practice. This property depends greatly on the implant conditions (in other words, whether the site is dry or bloody) and the temperature;
- manageability: the procedure for preparing the cement in the operating theatre must consist of simple, repeatable operations, and the operator's manual ability must not be a decisive factor.

Typically, cements used in operating theatres consist of two components, usually one solid and one liquid, which must be supplied in sterile packages (implying that the components must be able to withstand the sterilization conditions without losing their properties), and which must be mixable in the theatre according to a highly standardized and repeatable protocol.

The most widely used products are bone cements based on polymethyl methacrylate (PMMA), which have the advantage of allowing the cement to be conveniently prepared and used by surgeons in the operating theatre, and also of providing mechanical stabilization effects a few minutes after implantation. Acrylic-based cements therefore eliminate many of the aforementioned difficulties, since they are rather simple to mix, the injectability and cohesion of the end product are optimal, and they have good mechanical properties enabling the vertebra to be rapidly stabilized.

However, the use of PMMA is associated with a number of problems which may limit its use, especially in the case of patients who are young and/or still physically active.

One of the most significant problems is the large exothermic effect associated with the polymerization of acrylates, which may cause the development of temperatures of up to more than 100°C at the interface with the surrounding bone, creating a high risk of local bone necrosis (10 - 13).

Additionally, PMMA is completely bioinert and non-porous, so that the injected substance remains in the vertebral body as a foreign object which is discontinuous with the surrounding bone, and adversely affects the general functionality of the spinal column. This is because PMMA has high compression strength but excessive rigidity as compared with bone, and therefore the physical discontinuity with the surrounding bone leads to a risk of fractures affecting the adjacent vertebral bodies (14 - 16).

The porosity of the cement, that is to say its capacity to be permeable to cells, is an important requirement for efficient bone regeneration, since it allows the bone to form not only at the interface with the cement but also within the cement.

Acrylic cements, as mentioned above, do not promote bone formation and are not porous.

Another desirable characteristic for efficient bone regeneration is the bioresorbability of the cement; in other words, the cement should be resorbed progressively as the new bone is formed and penetrates.

With the cements described above, therefore, good mechanical stabilization of the damaged vertebral body is achieved, but the body is not regenerated.

The absence of regeneration is not particularly significant for an elderly patient or one with limited activity, for whom the primary aim is to limit the length of his stay in hospital and his immobility. However, for a physically active person, the physical stressing of a vertebra containing a rigid object within it, which acts as a foreign body, may result in secondary fractures and other complications. For a population of persons who are young, or in any case still physically active, always present in the greater number even among elderly people, these secondary complications and risks are unacceptable.

Solutions for regenerating bone and restoring correct spinal function are therefore highly sought.

It is particularly sought a material which promotes bone formation not only outside but also inside the cement, and which is therefore a material characterized by high porosity while also being bioresorbable.

Much research has been carried out recently on the development of calcium phosphate cement, using self-hardening mechanisms initiated by practically isothermic reactions of transformation of calcium phosphates into apatite or brushite, making it possible to obtain cements having a very similar composition to that of bone (10, 17, 18).

However, when calcium phosphates are used the cement is less plastic, making it necessary to include additives to improve its slip. Furthermore, the mechanical properties remain poorer than those of acrylic cements, partly because hardening takes place by the formation of a new phase (hydroxyapatite) which is hardened by the physical interpenetration of its elongated particles, instead of by polymerization. However, this is a positive aspect, since an isothermal hardening reaction does not damage the surrounding tissue. A further shortcoming of the apatite cements that have been developed hitherto is that their micrometric porosity is inadequate for cellular penetration of the new bone tissue; this limits osteointegration and bioresorption, and true tissue regeneration is not achieved.

It would therefore be desirable to be able to achieve rapid penetration of new bone into the injected cement, in association with progressive resorption, thereby obtaining, in a relatively short time, mechanical stabilization of the bone cement construct which increases progressively and is increasingly biologically competent in mechanical and functional terms.

Hydroxyapatite (Ca₁₀(PO₄)₆(OH)₂) is a calcium phosphate considered to be one of the most useful materials as a bone replacement, since it is an essential component of the mineral/inorganic part of mammal bones. Physiological hydroxyapatite also contains a certain amount of ionic species substituted for the calcium and/or phosphorus, which have specific functions in relation to the biological processes, and which make the substance more resorbable, providing a greater capacity to stimulate the growth of new bone tissue. It is known, therefore, that the presence of these substituent ions is essential for the provision of good bone integration and regeneration.

There are some known calcium phosphate cements which allow bone penetration to a certain extent. However, as yet there are no bone cements which can allow bone penetration and are bioresorbable, thus promoting full regeneration of the vertebra.

These characteristics depend primarily on the composition of the apatite, that is to say the presence of substituent ions, but also on its porous structure which allows cellular penetration.

There are known cements composed of apatite substituted with various ions, however no osteoconductive and bioresorbable bone cements capable of regenerating damaged vertebral bodies are yet available on the market.

There are known methods for producing ion-substituted hydroxyapatite cements. In these methods, however, the substituent ions are used in the form of salts, and are mixed with commercial α-tricalcium phosphate (TCP: Ca₃(PO₄)₂), usually with a good degree of purity.

To summarize the above, the greatest difficulties in the development of compositions for use as injectable apatite cements with suitable properties for the support and regeneration of damaged vertebral bodies are due to the fact that the following properties are not simultaneously provided:
**i)** the capacity for osteogenesis, adequate osteointegration, and resorption (17);
**ii)** viscosity, injectability and optimal hardening times;
**iii)** suitable mechanical properties, especially in the long term, when the absence of bone penetration means that the bone cement construct is incapable of supporting physiological mechanical loads.

The presence of each specific ion in suitable amounts provides a particular contribution to the biological process of bone remodelling and regeneration, and has an effect on the solubility, resorption, stability and mechanical strength of the substituted hydroxyapatite. All the substituent ions that are naturally present in physiological hydroxyapatite therefore contribute to the determination of the specific biological characteristics of the bone tissue.

It is therefore highly desirable to have a synthetic material which reproduces the characteristics provided to the bone tissue by physiological apatite in such a way that fully biocompatible and biomimetic bone substitutes can be obtained.

For example, WO2007045954 describes hydroxyapatite multi-substituted with various ion species and its composition with a natural or synthetic polymer for correcting bone tissue defects. In particular, a hydroxyapatite comprising silicate and/or Sr²⁺ ions and an ionic species selected from carbonate, magnesium and/or mixtures thereof is described.

US2008260714 describes injectable bone cement with fibrin and an inorganic component, for example α-TCP substituted with Sr.

CN1799643 describes a bone cement comprising a polymer and α or β-TCP with substituted ions, in which the ions used include Sr and Si.

WO2012014172 describes magnetic hydroxyapatite in which the calcium ions are partially substituted by Fe²⁺ and Fe³⁺ ions.

There are also known tricalcium phosphates (TCP) substituted with Si and Sr for bone regeneration material (40) or α-TCP substituted with Si for use as bone cement (41).

However, the problem remains of providing an injectable, self-hardening and bioresorbable cement in which the calcium and/or the phosphorus of hydroxyapatite is substituted with strontium and/or silicon and/or magnesium ions, and which meets the requirements of ease of application in the operating theatre, has compatible hardening times, is osteo-integratable, and promotes osteogenesis.

Additionally, there are known to be critical problems for calcium phosphate cements (CPC) in respect of the prevention of solid-liquid separation during extrusion and the complete transfer of the cement through a syringe, which are basic requirements for the potential clinical use of a bone cement.

The above problems are resolved by a cement according to the present invention.

This is because the present invention provides a cement characterized by:
**i)** injectability according to clinical requirements;
**ii)** bioactivity, due to the presence of ions which make the cement more soluble and enable it to release these ions progressively;
**iii)** osteogenicity, osteoconductivity and bioresorbability, allowing a rapid formation of new bone and proliferation within the injected substance.

Owing to the simultaneous presence of all the properties described above, the cement according to the invention allows the regeneration of the damaged bony part, in particular a vertebra, enabling it to return to the condition preceding the damage.

### SUMMARY OF THE INVENTION

One object of the present invention is an αTCP-based injectable apatite cement which can be useful for bone regeneration, in particular for regenerative vertebroplasty and kyphoplasty and for pathologies characterized by losses of bone substance, in which the calcium in the tricalcium phosphate in the α phase is partially substituted with strontium and the phosphate in the α phase is optionally partially substituted with silicon, said cement comprising at least one polymer of natural origin and/or at least one bioerodible polymer and/or protein and/or plant polysaccharides, and, if necessary, additives for regulating the hardening time, and in which tricalcium phosphate in the β phase is also present in an amount kept within the range of 20% to 30% with respect to the total of α+β phases.

Another object of the present invention is said injectable apatite cement in which, in said tricalcium phosphate in the β phase, the calcium is partially substituted with strontium and/or magnesium and the phosphate is optionally partially substituted with silicon.

In the present description, the cement according to the invention is also referred to as "paste".

One object of the present invention is a process for the solid-phase preparation of said cement.

The cement according to the present invention is obtained by a novel solid-phase preparation method comprising the steps of:
a) adding strontium carbonate powder and optionally silica, to calcium carbonate and anhydrous dibasic dicalcium phosphate in powder form, and mixing in the dry state;
b) pressing the powders thus obtained, so as to produce pellets;
c) heating said pellets at temperatures above 1200°C in the case of strontium carbonate, or above 1000°C in the case of silica;
d) cooling at a cooling rate such that the β phase of the tricalcium phosphate is kept within the range of 20% to 30% with respect to the total of α+β phases;
e) grinding said pellets to form a powder;
f) optionally, adding glycerol in a powder to glycerol ratio variable from 0.1% to 10% w/w;
g) adding an aqueous solution of sodium hydrogen phosphate dihydrate containing at least one polymer of natural origin and/or at least one bioerodible polymer and/or proteins and/or plant polysaccharides in a concentration of between 0.1% and 50% by weight relative to said solution, and optionally additives for regulating the hardening times.

Preferably, strontium is present, in step a), in a Sr/(Ca+Sr) ratio of between 0.1% and 50% molar.

Preferably, the silicon is added in step a) in a Si/PO₄ ratio of between 0.1% and 20%.

Optionally, said process comprises a further step e^{I}) which comprises adding a α-TCP doped with silicon powder produced according to Mestres et al. (30), with a Si/PO₄ ratio of between 0.1% and 20%.

Optionally, said process comprises a further step e^{II}) of adding a powder of hydroxyapatite substituted with carbonate ions (CO₃)²⁻, in which the CO₃/PO₄ ratio is comprised between 0.1% and 20%, preferably not less than 8%.

Optionally, said process comprises a further step e^{III}) of adding a powder of hydroxyapatite bi-substituted with magnesium ions (Mg²⁺) and carbonate ions (CO₃)²⁻, in which the Mg/Ca ratio is comprised between 0.1% and 10%, preferably not less than 3%, and in which the CO₃/PO₄ ratio is comprised between 0.1% and 20%, preferably not less than 8%.

Optionally, said process comprises a further step e^{IV}) of adding a powder of hydroxyapatite bi-substituted with silicate ions (SiO₄⁴⁻) and carbonate ions (CO₃)²⁻, in which the Si/P ratio is comprised between 0.1% and 15%, preferably not less than 1%, and in which the CO₃/PO₄ ratio is comprised between 0.1% and 20%, preferably not less than 8%.

Optionally, said process comprises a further step e^{V}) of adding a powder of hydroxyapatite bi-substituted with strontium ions (Sr²⁺) and carbonate ions (CO₃)²⁻, in which the Sr/(Ca+Sr) ratio is comprised between 0.1% and 50%, preferably not less than 8%, and in which the CO₃/PO₄ ratio is comprised between 0.1% and 20%, preferably not less than 8%.

Optionally, said process comprises a further step e^{VI}) of adding superparamagnetic hydroxyapatite substituted with Fe²⁺ and Fe³⁺ ions (see WO2012014172).

These hydroxyapatites can be added in a proportion of 0% to 30% w/w to the TCP powder.

Regarding the superparamagnetic hydroxyapatite substituted with Fe²⁺ and Fe³⁺ ions (step e^{VI}), the quantity may vary from 0% to 50% w/w.

Each of said steps e^{I)}-e^{VI}) can be executed independently, either individually or in any desired combination, without the need to follow any particular sequence.

In step c), the pellets are heated at a temperature of more than 1200°C, preferably more than 1400°C, for a period of between 1 and 20 hours, preferably for one hour. In the case of Si substitution, the temperature may be more than 1000°C.

In step d), the cooling is preferably carried out rapidly in order to stabilize the αTCP partially against the recrystallization of the polymorphous βTCP. The cooling is controlled in such a way that the quantity of the β form is kept within the range of 20% to 30% of the total of phases α+β. Preferably, the cooling is carried out for a period of between 0.01 (in the case of cryogenic cooling) and 1 minute. Cooling is carried out down to a temperature below that required to prevent or at least limit the crystallization of the polymorphous βTCP.

In step e), grinding is preferably carried out using a planetary mill, or more preferably using zirconia grinding media. Grinding takes place for a sufficient time to obtain adequate surface reactivity in relation to water, thus achieving hardening times suitable for clinical practice, that is to say preferably in the range from 5 to 20 minutes.

In step f), which is optional, glycerol may be added in order to regulate the hardening times of the paste produced in the subsequent steps. In this specific case, glycerol is useful for increasing the hardening (setting) time.

In step g), the added aqueous solution preferably comprises phosphate ions in a concentration of between 2% and 7%, preferably 5%.

The liquid to powder ratio is adjusted (in the range from 0.3 to 0.6 ml/g, preferably 0.5 ml/g) to provide optimal properties in terms of injectability, cohesion, hardening times and mechanical strength.

The polymer of natural origin used in step g) is preferably selected from the group consisting of alginate, gellan, cellulose, collagen, gelatin, chitosan, xanthan and soya. The bioerodible polymer is preferably selected from the group consisting of polycaprolactone, polylactic acid, polyacrylic acid and polyvinyl alcohol.

The polymers may be added in step g) in a quantity of between 0.1% and 50% by weight; preferably, alginate is added in a proportion of 2%.

As in the preceding step f), in step g) it is possible to include additives, if desired, to regulate the hardening times, which may be useful in the case of particularly complex compositions where the setting times are found to be excessively long. In one embodiment of the invention, these additives are citric and tartaric acid, used in concentrations of between 1% and 10% w/w and 1% and 50% w/w respectively.

The process according to the invention can be used to produce an injectable paste which can be transformed into hydroxyapatite substituted with various ions, as described above, in which both the α and the β form of the tricalcium phosphate are present. This makes it particularly suitable for use for bone regeneration, in particular because of the presence of biomimetic hydroxyapatite and the β-TCP phase, which is bioactive and bioresorbable.

A further advantage of the injectable paste according to the present invention is that it has adequate cohesion, thus preventing solid-liquid separation during extrusion, and is fully injectable.

Another object of the present invention is an injectable cement obtained by the process described above.

The obtained paste can be injected *in vivo,* where it can harden and act as a bone cement, in particular for vertebroplasty and kyphoplasty, thanks to its osteogenic and osteoconductive properties; it is also able to be progressively resorbed *in vivo.*

Consequently a further object of the present invention is the use of the aforesaid paste for bone regeneration.

In particular, the paste has a high regenerative capacity and excellent mechanical properties which make it particularly suitable for use as a bone replacement.

More particularly, one object of the invention is the use of the paste in vertebroplasty and kyphoplasty.

Vertebroplasty is a procedure for treating pathological vertebral fractures by injecting a biocompatible cement into the body of the vertebra.

Kyphoplasty is a procedure in which a balloon is introduced into the collapsed vertebral body to dilate it and return it to the normal height before the cement is injected.

Given the characteristics of bioresorbability and osteoregeneration, one object of the present invention is the use of the paste described herein in vertebroplasty and kyphoplasty, in particular in osteoporosis patients or in young and/or physically active patients, even more particularly if these patients are suffering from vertebral fractures in which acrylic cements are contraindicated.

Another object of the invention is the use of the paste in orthopaedics for treating losses of spongy bone substance in the knee (particularly in the distal femur or the tibial plateau) or in the wrist or ankle, or in the proximal humerus (shoulder) or the heel, due to metabolic pathologies such as osteoporosis, or for treating avascular osteonecrosis in the head of the femur, or for the augmentation of long bones such as the humerus, or for tibioplasty procedures, or for treating dental or maxillofacial bone defects, or, more generally, for treating bone regions characterized by tissue loss or weakening as a result of injuries, pharmacological treatments, tumours, infections or mobilization of joint prostheses, or osteoporosis.

### DETAILED DESCRIPTION OF THE INVENTION

### Figures

Figure 1. Macroscopic analysis of explants embedded in methyl methacrylate. On the left: cement containing 2% strontium, i.e. Sr/(Ca+Sr) = 2 mol%; on the right: KyphOs FS™.
Figure 2. X-ray image of the two materials implanted in the distal metadiaphyseal region of rabbit femurs: a) cement containing 2% strontium, i.e. Sr/(Ca+Sr) = 2 mol%; b) KyphOs FS.
Figure 3. Histologies according to the present invention (1.25 X (a) and 20 X (b-c-d)).
Figure 4. a) Compressive strength of a cement with Sr/(Ca+Sr) = 2% after immersion in SBF at 37°C for various immersion times; b) Stress curves of the same cement with and without alginate.

### Description of the invention

Because of its metastability at room temperature, α-tricalcium phosphate (a-TCP) in contact with water is progressively transformed into calcium-deficient hydroxyapatite (HA) with an elongated nanometric morphology.

In particular, α-TCP phases ion-substituted with strontium and/or silicon are transformed into HA in a similar manner, transferring the ions initially present into the HA; in particular, the strontium-substituted HA (Sr-HA) is a biomaterial which, owing to its close resemblance to the mineral phase of bone and the presence of strontium, has a currently recognized efficacy in promoting bone formation while its resorption is limited, which is also a desired effect, especially in the case of osteoporosis or other pathologies resulting in compromised physiological turnover of the bone (3 - 9).

The calcium and/or phosphorus can be substituted in the paste object of the present invention by one or more of the following ions:
**1) strontium,** which is a recognized therapeutic anti-osteoporotic agent, capable of rebalancing the physiological turnover of bone and increasing its mass and density (19);
**2) silicon,** which is a recognized agent active in bone neoformation, and is particularly capable of stabilizing the neoformed bone matrix (20);
**3) magnesium,** which is an element associated with the first stages of the bone neoformation process and promotes the formation of new mineral phase nuclei (21);
**4) carbonate,** which is an element present in young bone and which, by increasing the solubility of apatite, promotes its dissolution and the bioavailability of the constituent ions (22);
**5) iron** which, in specific oxidation states and relative quantities in the apatite lattice, imparts intrinsic superparamagnetic properties to the apatite (23). The superparamagnetic properties allow remote activation/inactivation of the cement (that is to say, its magnetization/demagnetization) by an external magnetic signal, to obtain advanced functionality such as the "on demand" controlled reclamation and/or release of bioactive molecules such as drugs or growth factors (24).

The substituent ions are preferably present in the following molar ratios relative to the original calcium or phosphate ions: 0.1% to 50% strontium to calcium, 0% to 20% silicon to phosphate, 0% to 10% magnesium to calcium, 0% to 15% carbonate to phosphate, and 0% to 30% iron to calcium.

The present invention has the following advantages over the prior art in this field:
**1)** the ion-substituted calcium phosphate precursors (i.e. Ca₃(PO₄)₂ (TCP) in its two polymorphs α and β) are produced by synthesis, by contrast with the usual procedure in which the substituent ions are present in salts (e.g. SrCO₃, SrHPO₄) mixed appropriately with commercial αTCP, to which are optionally added powders of hydroxyapatite substituted with magnesium and/or carbonate and/or silicon and/or strontium, and/or with superparamagnetic iron-substituted hydroxyapatite. As a result of the addition and mixing with an aqueous solution of Na₂HPO₄ which also contains an aqueous solution of natural and/or bioerodible polymers, a mixture is produced which is self-hardening at ambient temperature. During the process of the hardening of the cement in vivo, the fraction of α polymorph is progressively transformed into hydroxyapatite (HA), transferring into its crystal structure the ions contained in it (strontium and/or silicon), which are then made bioavailable by the progressive dissolution of the HA, while the β fraction, inert in respect of the transformation process, can be progressively resorbed (25), thus promoting osteointegration while simultaneously releasing any calcium substituent ions which have been incorporated into its crystal structure during the synthesis process, in addition to the calcium itself and the phosphorus. Any addition of powders of hydroxyapatite substituted with magnesium and/or carbonate and/or silicon and/or strontium and/or iron also provides a source of ions which are biologically competent for bone regeneration and/or functional for drug delivery applications. For example, in the administration of antibiotics, growth factors, or the like, the presence of progressively bioerodible natural polymers promotes the slip properties which affect the injectability, the bio-erosion, and the penetration and integration of new bone until the cement is completely replaced with the neoformed bone.
**2)** The presence of multiple ions which are biologically competent for the induction and stabilization of the bone matrix, made bioavailable by the solubility of the cement in vivo.
**3)** The possible specific presence of partially iron-substituted hydroxyapatite with superparamagnetic properties and the transfer of this property to the cement, which can serve as an intelligent drug delivery system. This type of system is capable of responding and activating itself in response to specific stimuli (e.g. environmental stimuli of temperature and/or pH, or from an external signal).
**4)** The use of natural and/or bioerodible polymers and/or protein of plant origin, for promoting injectability, cohesion and osteoconductivity of the injected cement.
**5)** The variety of possible custom-designed applications. Vertebroplasty and kyphoplasty are procedures designed to restore the bearing capacity of vertebral bodies damaged as a result of various pathologies and/or injuries. In this context, the novel cement has regenerative capacities, unlike the current available solutions, and has a composition that can be adapted to different clinical pictures, including those related to the patient's age. More precisely, the various ions present in the cement, in their position as ions substituting for the calcium and/or phosphorus in biomimetic and bioresorbable apatite phases, and having a different effect on bone regeneration, can be dosed suitably in response to different therapeutic requirements.

### EXAMPLES

### Example 1

### Synthesis of the inorganic precursors

### Preparation of the cement

### 1) α-TCP substituted with strontium and/or silicon

Powders of αTCP strontium-substituted with different quantities of strontium were synthesized by a solid state reaction of stoichiometric quantities of calcium carbonate (CaCO₃, Carlo Erba, Italy), anhydrous dibasic dicalcium phosphate (CaHPO₄, Sigma Aldrich) and strontium carbonate (SrCO₃, Carlo Erba, Italy), according to the reaction:

x SrCO₃ + (1-x) CaCO₃ + 2CaHPO₄ → Ca₃₋ₓSrₓ(PO₄)₂ + H₂O + CO₂

In a similar way to that described above, SiO₂ powder was added to the aforesaid reactants in variable amounts, namely an Si/PO₄ ratio from 0% to 20%, and preferably not less than 2%, to produce α-TCP phases co-substituted with strontium and silicon.

The powders were initially mixed in the dry state for an optimal time (preferably 30 minutes), and were then pressed uniaxially (preferably at 15 MPa) to form pellets which were finally treated at temperatures of more than 1250°C (preferably 1400°C) for a period of between 1 and 20 hours (preferably 1 hour).

After the high-temperature treatment, the pellet was subjected to rapid cooling, being taken quickly out of the hot furnace, to partially stabilize the αTCP against the recrystallization of the βTCP polymorph.

After cooling, the end product was ground and screened to less than 150 µm and then ground again (Pulverisette 6 classic line, Fritsch, Germany) at 400 r.p.m. for optimal periods (from 0 to 200 minutes, preferably 50 minutes) so as to obtain adequate reactivity in contact with liquid media, in other words hardening times suitable for clinical practice, that is to say less than 15 minutes. The liquid/solid ratio is specified in values from 0.3 to 0.6 ml/g (preferably 0.43) to optimize the injectability and mechanical properties.

The particle size distribution was evaluated by means of a SediGraph (FlowSorb II 2300, Micromeritics, USA).

An aqueous solution of disodium phosphate dihydrate (Na₂HPO₄·2H₂O, Fluka) in the range from 2% to 7% (preferably 5%) was prepared and mixed in various concentrations with the calcium phosphate precursor to form a self-hardening paste.

The liquid to powder ratio was adjusted on the basis of the characteristics of the inorganic part (in the range from 0.3 to 0.6, preferably 0.43) to provide excellent properties in terms of injectability, cohesion, hardening times and mechanical strength.

A quantity of polymer equal to 2% by weight, particularly alginate (Alginic Acid Sodium Salt from Brown Algae, Sigma Aldrich), was incorporated into the aqueous solution.

The properties of the cement were validated by a robust chemical, physical, morphological, mechanical and biological characterization *in vitro* and *in vivo.* In particular, it was found, by X-ray diffraction spectrum analysis and by comparison with the existing literature (26 - 29), that the introduced strontium entered the crystal structure of the TCP (α and β) in substitution of the calcium in equal quantities to those introduced, without the formation of any other secondary phase. The only additional phase present was the βTCP, which is the polymorph of TCP stable at ambient temperature and is a bioactive and bioresorbable biomaterial. The effect of the prolonged grinding on the setting times was evaluated, in order to ascertain the most suitable values for the practical clinical use of the cement. The setting times were evaluated using Gillmore needles, according to the ASTM C266-99 directives.

The initial in vitro biological tests showed that the cements that were developed were biocompatible and induced cell proliferation on the surface, while the initial in vivo tests showed that the cement was inhabited and penetrated by new organized bone even within one month of implantation in a rabbit, which is better than the performance of a commercial cement used in kyphoplasty (KyphOs™, Medtronic).

The results were confirmed by follow-up tests at three months and by tests conducted on ovariectomized mice.

### 2) α-TCP substituted with silicon

Commercial powders of hydroxyapatite and SiO₂ were mixed according to the procedure described by Mestres et al. (30) in various SiO₂/(HA + SiO₂) concentrations in a range from 0.1% to 20% by weight, and were then treated by prolonged grinding (Pulverisette 6 classic line, Fritsch, Germany) at 400 r.p.m. for optimal periods of 10 to 60 minutes, preferably 30 minutes. The resulting powder was sintered at temperatures of 1000°C to 1500°C, preferably 1250°C, for a period of 1 to 5 hours (preferably 2 hours), followed by spontaneous cooling.

### 3) Synthesis of hydroxyapatite substituted with CO₃ and/or co-substituted with Mg and CO₃ and/or co-substituted with SiO₄ and CO₃ and/or co-substituted with Sr and CO₃ (5, 31, 32, 38, 39)

Powders of hydroxyapatite substituted with carbonate ions and/or co-substituted with magnesium and carbonate ions and/or co-substituted with silicate and carbonate ions and/or co-substituted with strontium and carbonate ions were synthesized according to the procedures described in (39), (38), (32) and (5) respectively, so as to produce quantities (as percentages by weight relative to the hydroxyapatite powder) of CO₃ ions from 0.1% to 10% w/w, preferably not less than 3% w/w; of magnesium ions from 0.1% to 15% w/w, preferably not less than 1% w/w; of silicon from 0.1% to 5% w/w, preferably not less than 0.4% w/w; and of strontium ions from 0.1% to 20% w/w, preferably not less than 5% w/w.

### 4) Synthesis of iron-substituted hydroxyapatite with intrinsic magnetic properties

Nanoparticles of intrinsically magnetic hydroxyapatite were synthesized by the method also described in Tampieri et al (23).

### Formulation of the final inorganic precursor

The inorganic component of the cement according to the present invention was produced using precursor 1) on its own or mixed with precursor 2) and/or with precursor 3) and/or with precursor 4).

### Production of the injectable paste

To produce the cement, the processed powder was mixed with an aqueous solution of Na₂HPO₄ in a concentration from 2% to 7% (preferably 5%). A solution of natural polymers (e.g. sodium alginate and/or gellan and/or collagen and/or cellulose and/or chitosan) and/or bioerodible polymers (polyvinyl alcohol and/or polylactic acid and/or polyacrylic acid and/or polycaprolactone), preferably sodium alginate (Alginic Acid Sodium Salt from Brown Algae, code no. 71238, Sigma Aldrich), was added to the Na₂HPO₄ solution in variable proportions from 0.1% to 6% (preferably 2%). At this point the paste was ready for injection.

### Example 2 - In vivo analysis

### Materials and Methods

The osteoconductivity of a cement with Sr/(Ca+Sr) = 2%, prepared according to the preceding example, was evaluated by implantation into metadiaphyseal regions of rabbit femurs according to the protocols described in (34-36), in comparison with a commercial bone cement widely used in vertebroplasty and kyphoplasty (KyphOs FS™ - Medtronic). These two cements were implanted into 18 male NZW rabbits weighing approximately 3 kg.

The animals were sacrificed one month after the operation and the explants were subjected to macroscopic, X-ray and histological analysis.

The macroscopic analysis was conducted to determine the existence or position of defects in the bone tunnel and the presence of any infections, deformities or fractures. The X-ray analysis analysed the amount of bone loss, the radiopacity of the material and/or resorption or substitution, perimetric radiolucency, heterotopic ossification, and any deformity or fracture.

For the histological analysis, the specimens were fixed in a 4% solution of paraformaldehyde in a 0.1 M phosphate buffer solution, pH 7.4, at 4°C for 12 hours. They were then embedded in methyl methacrylate (Technovit 7200, Bioptica), cut transversely into thin sections (< 20 mm) with a cutting system (Exact, Bioptica), and fixed with haematoxyline and eosine and toluidine blue. Three central sections of each specimen were examined and analysed.

The microscopic examination was carried out using an optical microscope (Carl Zeiss Axioscop 40) connected to a video camera (Axiocam ICC 3 Zeiss) and dedicated image acquisition software (Axiovision 4.8).

The microscopic bone-material contact interface was analysed; bone growth and bone penetration were evaluated.

Histomorphometric analysis was performed to evaluate the bone remodelling around the implanted biomaterial. Standard terms and nomenclature for the histomorphometric analysis of the bone were used in accordance with ASBMR Histomorphometry Nomenclature Committee JBMR 2013 (37).

### Results

The cements were evaluated after a follow-up of one month.

### Macroscopic analysis

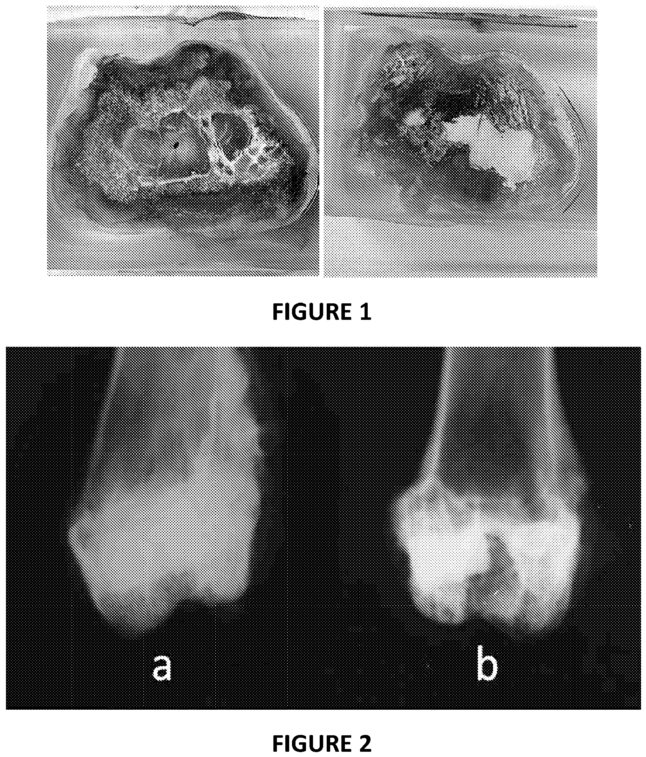

The macroscopic analysis of the two specimens showed no difference. There were no fractures, infections, or bone healing defects. The macroscopic appearance of the two cements was different: the present invention was apparently well osteointegrated, less dense and more similar in qualitative terms to bone (Figure 1, left). The KyphOs cement also appeared to be osteointegrated but appeared denser and more compact (Figure 1, right).

### X-ray examination

X-ray examination showed that both implanted materials had a greater density than bone. No traumatic or iatrogenic fractures were detected, and no occurrence of osteolysis or radiolucency was detected around the perimeters of both materials. Smaller, and comparable, heterotopic ossifications were found (Figure 2).

### Optical microscopy

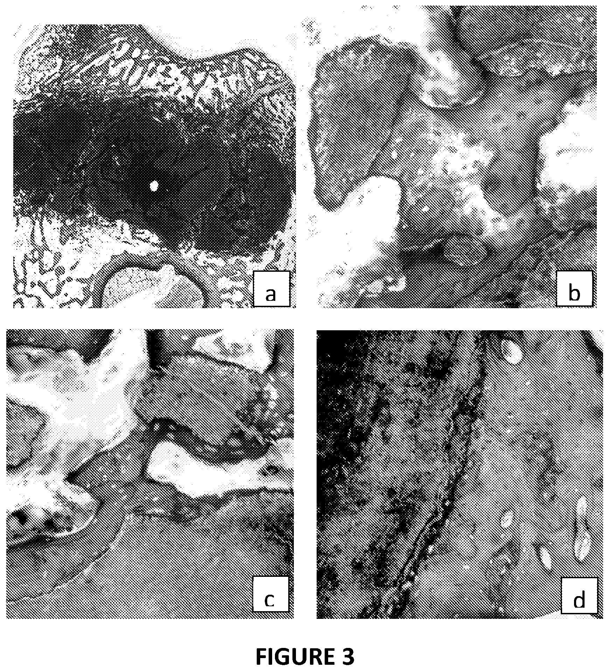

Good osteointegration was found in both materials. Apparently, the quality and quantity of the bone in our invention was higher than in the control. In particular, our invention showed a greater quantity of mature bone, with good adhesion between the neoformed bone and the cement. Extensive bone growth and high penetration of new bone were found in our invention (Figure 3). The control group showed similar, but less evident, results.

### Histomorphometry

The histomorphometric analysis was performed to evaluate the bone remodelling around the implanted biomaterials and to quantify the formation of new bone. All evaluations were standardized according to ASBMR 2013 (37).

The most important measurements made were as follows (Table 1):
- Total area of implanted cement (TM-Ar);
- Pore area of implanted cement (MPo-Ar);
- The real area of the implanted material, determined by subtracting the area of the pores of the implanted cement from the total area of the implanted cement **(RM-Ar);**
- Perimeter of the material (M-Pm)
- The length of contact between material and bone (BMC-Le), which is the total length of the bone in contact with the material.

**Table 1. Histomorphometric analysis**

| | Sr-HA CEMENT | KyphOS FS™ |
|---|---|---|
| **TM-Ar (µ²)** | 102,168,471.2 | 67,019,076.8 |
| **M-PO-Ar (µ²)** | 588,031.9 | 747,794.7 |
| **RM-Ar (µ²)** | 101,580,439.3 | 66,271,282.03 |
| **M-Pm (µ)** | 63,110.5 | 54,870.3 |
| **BMC-Le (µ)** | 29,310.5 | 21,103.3 |
| **MPoPe** | 0.6% | 1.1% |
| **BMCR** | 46.4% | 38.5% |
| **BPR-Le** | 85.4% | 22.1% |

From these measurements, some parameters were derived which indicate the osteoconductive behaviour of the materials:
- Porosity percentage of the material (MPoPe), which is the ratio between the total area of the material (TM-Ar) and the pore area (MPo-Ar). This is indicative of macroporosity, and therefore of the potential to accept neoformed bone ("bone ingrowth").
- Bone-biomaterial contact rate (BMCR), which is the percentage of bone-biomaterial contact (BMC-Le) relative to the total length of the material (M-Pm). This is indicative of the bone growth, and consequently of the osteoconductivity.
- The bone penetration length rate (BPR-Le) is an index of the bone penetration into the material and also an index of osteointegration and bone growth. It provides an indication of the rate of osteoconduction at defined times, by measuring the distance of penetration of the bone into the pores of the material from the periphery towards the centre.

BMC-Le and BMCR are two indices of osteointegration (or osteoconductivity) of the cement, and are higher in our invention than in the control. To make it significant, BMC-Le was correlated with BMCR, which is a derived index of bone osteointegration. The BMCR of our invention is significantly higher (46.4%) than in the control (38.5%); this confirms that our invention induces greater bone growth than the control.

In our invention, MPoPe is approximately 0.6%, as against 1.1% in the control, and therefore the latter exhibits greater porosity after one month.

Another important index of osteoconductivity is the bone penetration rate (BPR-Le), in the sense of a measurement of the length of bone penetration from the external part to the internal part of the material. Our invention induces a higher rate of penetration (85.4%) than the control (22.1%), which is significantly higher.

These results demonstrate that our invention exhibits excellent behaviour as an injectable cement and better osteogenic and osteoconduction properties than the control.

### Example 3

### Compressive strength

### Materials and Methods

Cylindrical specimens were produced for the evaluation of the mechanical properties by injecting the paste into suitable moulds for the specification of the measurement times. The compressive strength and the Young's modulus of hardened cement specimens were evaluated after 1, 3, 5, and 30 days of immersion in simulated body fluid (SBF) at 37°C (12 specimens for each measurement time) according to the ISO 9917 standard, using a universal examination machine (MTS Insight 5, Minnesota, USA). The expression "simulated body fluid" (SBF) denotes a buffered aqueous solution at a pH of about 7, containing various ions present in physiological liquids. It is commercially available and was prepared according to the instructions given in Oyane A, Kim H-M, Furuya T, Kokubo T, Miyazaki T, Nakamura T (2003) Preparation and assessment of revised simulated body fluids. Journal of Biomedical Materials Research Part A 65A: 188-195. The tests were conducted with a crosshead speed of 0.5 mm/min. Young's modulus was approximated by calculating the slope of the stress curve obtained during the compression test in its elastic region.

### Results

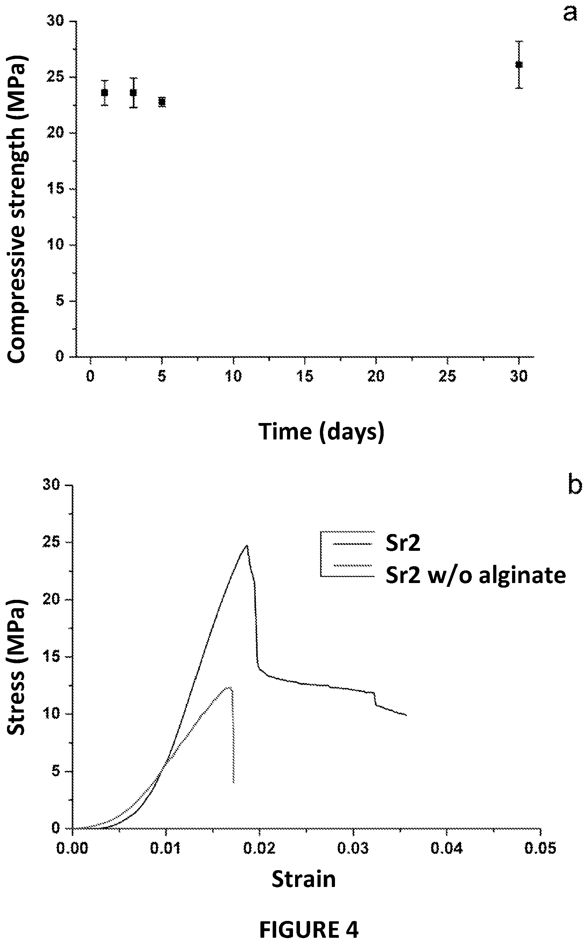

The Sr-substituted cement with Sr/(Ca+Sr) = 2% exhibited a compressive strength σ of 24.5 ± 1.6 MPa and a Young's modulus E of 2.3 ± 0.6 GPa, after one day of immersion in SBF at 37°C, and was stable up to one month of immersion (Figure 4a). The same cement exhibited better breaking resistance under uniaxial compression, compared with the alginate-free cement (Figure 4b), which exhibited a compressive strength σ of 12.2 ± 1.2 MPa which was statistically significantly lower (p<0.01) and a Young's modulus E of 1.1 ± 0.1 GPa following the same immersion conditions.

As a control, a polymer-free formulation was prepared, with the same liquid to powder ratio, in order to compare the mechanical performance: in addition to incomplete injectability, the polymer-free cement exhibited a lower ultimate compressive strength and rapid collapse. This suggests that the incorporation of alginate may reinforce the structure of the cement by a fissure bonding mechanism, as proposed previously (42), thus preventing both the abrupt collapse and the fragmentation of the cement *in vivo.*

### References

1) N. B. Watts, S. T. Harris and H. K. Genant, Treatment of Painful Osteoporotic Vertebral Fractures with Percutaneous vertebroplasty or kyphoplasty. 2001; 12(6): 429-437.
2) D.G. Poitout, Biomechanics and Biomaterials in Orthopedics, Springer 2004, p.86
3) E. Landi, A. Tampieri, G. Celotti, S. Sprio, M. Sandri, G. Logroscino, Sr-substituted hydroxyapatites for osteoporotic bone replacement, Acta Biomater 3 (2007) 961-969
4) M. Schumacher, A. Henß, M. Rohnke, M. Gelinsky, A novel and easy-to-prepare strontium(II) modified calcium phosphate bone cement with enhanced mechanical properties, Acta Biomater 9 (2013) 7536-7544
5) E. Landi, S. Sprio, M. Sandri, G. Celotti, A. Tampieri. (2008) Development of Sr and CO3 co-substituted hydroxyapatites for biomedical applications. Acta Biomater 4, 656-663.
6) S.J. Saint-Jean, C.L. Camirè, P. Nevsten, S. Hansen, M.P. Ginebra, Study of the reactivity and in vitro bioactivity of Sr-substituted α-TCP cements, J Mater Sci: Mater Med 16 (2005) 993-1001.
7) Pina S, Torres PM, Goetz-Neunhoeffer F, Neubauer J, Ferreira JMF. Newly developed Sr-substituted αTCP bone cements. Acta Biomater 2010 6: 928-935.
8) Bigi A, Boanini E, Capuccini C, Gazzano M. Strontium-substituted hydroxyapatite nanocrystals. 2007 Inorg Chem Acta 360: 1009-1016.
9) K.S. TenHuisen, P.W. Brown, Formation of calcium-deficient hydroxyapatite from α-tricalcium phosphate, Biomaterials 19 (1998) 2207-2217
10) Bohner M. Calcium orthophosphates in medicine: from ceramics to calcium phosphate cements. Injury Int J Care Injured 2000; 31: S-D37-47
11) S.M. Belkoff, S. Molloy. Temperature Measurement During Polymerization of Polymethylmethacrylate Cement Used for Vertebroplasty, Spine. 28(14): 1555-1559, 2003.
12) Po-Liang Lai, Ching-Lung Tai, Lih-Huei Chen and Nai-Yuan Nien, Cement leakage causes potential thermal injury in vertebroplasty, BMC Musculoskeletal Disorders 2011, 12: 116.
13) H. Deramond, N.T. Wright, S.M.Belkoff, Temperature elevation caused by bone cement polymerization during vertebroplasty. Bone Vol. 25, No. 2, Supplement. 1999: 17S-21S.
14) S. Becker, D. Dabirrahmani, M. Hogg, R. Appleyard, G. Baroud, M. Gillies, Disadvantages of Balloon Kyphoplasty with PMMA - a Clinical and Biomechanical Statement, J MINER STOFFWECHS 2011; 18 (Supplement 1).
15) Berlemann U., Ferguson S.J., Nolte L.P., Heini P.F. Adjacent vertebral failure after vertebroplasty. A biomechanical investigation. J Bone Joint Surg Br 2002; 84: 748-52.
16) G. Lewis, Injectable Bone Cements for use in vertebroplasty and kyphoplasty: state-of-the-art review. J Biomed Mater Res Part B: Appl Biomater 76B: 456-468, 2006.
17) M. Bohner, G. Baroud, Injectability of calcium phosphate pastes, Biomaterials 26 (2005) 1553-1563.
18) M. Espanol, R.A. Perez, E.B. Montufar, C. Marichal, A. Sacco, M.P. Ginebra, Intrinsic porosity of calcium phosphate cements and its significance for drug delivery and tissue engineering applications. Acta Biomater. 2009: 2752-62.
23) Tampieri A, D'Alessandro T, Sandri M, Sprio S, Landi E, Bertinetti L, Panseri S, Pepponi G, Goettlicher J, Bañobre-López M, Rivas J. (2012) Intrinsic magnetism and hyperthermia in bioactive Fe-doped hydroxyapatite. Acta Biomater 8: 843-851.
26) Bigi A, Foresti E, Gandolfi M, Gazzano M, Roveri N. isomorphous substitutions in β-tricalcium phosphate: the different effects of zinc and strontium. J Inorg Biochem 1997 66: 259-265.
27) Mathew M, Schroeder LW, Dickens B, Brown WE. The crystal structure of α-Ca3(PO4)2. Acta Cryst. (1977). B33, 1325-1333.
28) Yashima M, Sakai A, Kamiyama T, Hoshikawa A. Crystal structure analysis of β-tricalcium phosphate Ca3(PO4)2 by neutron powder diffraction (2003) 175(2): 272-277.
29) Rodriguez-Lorenzo LM, Hart JN, Gross KA. Structural and Chemical Analysis of Well-Crystallized Hydroxyfluorapatites 2003 J Phys Chem B. 107: 8316-8320.
30) Mestres G, Le Van C, Ginebra M-P. Silicon-stabilized α-tricalcium phosphate and its use in a calcium phosphate cement: Characterization and cell response. Acta Biomater 2012, 8: 1169-1179.
31) Landi E, Tampieri A, Celotti G, Sprio S, Pressato D, De Luca C. WO2007045954: A plurisubstituted hydroxyapatite and the composite thereof with a natural and/or synthetic polymer, their preparation and uses thereof. PCT/IB2006/002844, 2006.
32) Sprio S, Tampieri A, Landi E, Sandri M, Martorana S, Celotti G, Logroscino G. (2008) Physico-chemical properties and solubility behaviour of multi-substituted hydroxyapatite powders containing silicon. Mater Sci Eng C 28, 179-187.
33) Tampieri A, Landi E, Sandri M, Pressato D, Rivas Rey J, Banobre Lopez M, Marcacci M. WO2012014172: Intrinsically Magnetic Hydroxyapatite. PCT/IB2011/053362, 2010.
34) Landi E, Logroscino G, Proietti L, Tampieri A, Sandri M, Sprio S. Biomimetic Mg-substituted hydroxyapatite: from synthesis to in vivo behaviour. J Mater Sci Mater Med. 2008;19:239-47.
35) Barkarmo S, Wennerberg A, Hoffman M, Kjellin P, Breding K, Handa P, et al. Nano-hydroxyapatite-coated PEEK implants: a pilot study in rabbit bone. J Biomed Mater Res A. 2013;101:465-71.
36) Landi E, Tampieri A, Celotti G, Belmonte MM, Logroscino G. Synthetic biomimetic nanostructured hydroxyapatite. Key Eng Mat. 2005;284-286:949-52.
37) Dempster DW, Compston JE, Drezner MK, Glorieux FH, Kanis JA, Malluche H, et al. Standardized nomenclature, symbols, and units for bone histomorphometry: a 2012 update of the report of the ASBMR Histomorphometry Nomenclature Committee. J Bone Miner Res. 2013;28:2-17.
38) Landi E, Sprio S, Sandri M, Tampieri A, Bertinetti L, Martra G. (2008) Development of Multisubstituted Apatites for Bone Reconstruction. Key Eng. Mater 361-363, 171-174.
39) Landi E, Tampieri A, Celotti G, Vichi L, Sandri M. Influence of synthesis and sintering parameters on the characteristics of carbonate apatite. Biomaterials 25 (2004) 1763-1770.
40) Bandyopadhyay A, Petersen J, Fielding G, Banerjee S, Bose S. ZnO, SiO2, and SrO doping in resorbable tricalcium phosphates: Influence on strength degradation, mechanical properties, and in vitro bone-cell material interactions. J Biomed Mater Res B Appl Biomater. 2012 Nov;100(8):2203-12.
41) Reid JW, Tuck L, Sayer M, Fargo K, Hendry JA. Synthesis and characterization of single-phase silicon-substituted alpha-tricalcium phosphate. Biomaterials. 2006 May;27(15):2916-25.
42) Liu WZ, Zhang JT, Weiss P, Tancret F, Bouler JM (2013) The influence of different cellulose ethers on both the handling and mechanical properties of calcium phosphate cements for bone substitution. Acta Biomaterialia 9: 5740-5750.

## Claims

1. Injectable apatite cement, wherein calcium in the tricalcium phosphate in the α phase is partially substituted with strontium and the phosphate in the α phase is optionally partially substituted with silicon, said cement comprising at least one polymer of natural origin and/or at least one bioerodible polymer and/or proteins and/or vegetable polysaccharides, and optionally additives for regulating hardening time, and wherein tricalcium phosphate in the β phase is also present, said β phase of tricalcium phosphate is kept in the range of 20% to 30% with respect to the total of α+β phases.

2. Cement according to claim 1, wherein in said tricalcium phosphate in the β phase, calcium is partially substituted with strontium and/or magnesium and phosphate is optionally partially substituted with silicon.

3. Cement according to anyone of claims 1-2, wherein the substituent ions are preferably present in the following ratios with respect to the original calcium or phosphate ions: from 0.1 to 50% of Strontium to Calcium, from 0 to 20% of Silicon to Phosphate, from 0 to 10% of Magnesium to Calcium, from 0 to 15% of Carbonate to Phosphate, from 0 to 30% of Iron to Calcium.

4. Cement according to anyone of claims 1-3, wherein said polymer of natural origin and/or at least one bioerodible polymer are present in a concentration comprised between 0.1 and 6% in weight, preferably 2%, and/or said proteins and/or said vegetable polysaccharides are present in a concentration from 0 to 50% in weight.

5. Cement according to anyone of claims 1-4, wherein said polymer of natural origin is selected from the group consisting of alginate, gellan, cellulose, collagen, gelatin, chitosan, xanthan and soy and said bioerodible polymer is selected from the group consisting of polycaprolactone, polylactic acid, polyacrylic acid and polyvinyl alcohol.

6. Cement according to anyone of claims 1-5, further comprising iron-substituted superparamagnetic hydroxyapatite.

7. Process for the preparation in solid phase of the cement of claims 1-6 comprising the steps of:
a) adding strontium carbonate powder and optionally silica to powdery calcium carbonate and anhydrous dibasic dicalcium phosphate and dry mixing;
b) pressing the obtained powders so as to produce pellets;
c) heating said pellets at temperatures above 1200°C in the case of strontium carbonate or above 1000°C in the case of silica;
d) cooling at a cooling rate such that the β phase of tricalcium phosphate stays in the range of 20% to 30% with respect to the total of α+β phases;
e) grinding said pellets so as to form a powder;
f) optionally adding glycerol in a powder to glycerol ratio ranging from 0.1% to 10% w/w;
g) adding an aqueous solution of sodium hydrogen phosphate dihydrate containing at least one polymer of natural origin and/or at least one bioerodible polymer and/or proteins and/or vegetable polysaccharides in a concentration comprised between 0.1 and 50% by weight relative to said solution, and optionally additives for regulating hardening times.

8. Process according to claim 7, wherein in said step a) strontium is present in a Sr/(Ca+Sr) ratio comprised between 0.1 and 50% molar.

9. Process according to anyone of claims 7-8, wherein silicon is added in step a) in a Si/PO₄ ratio comprised between 0.1 and 20%.

10. Process according to anyone of claims 7-9, comprising a further step e^{I}) wherein a silicon doped α-TCP powder is added.

11. Process according to claim 10, wherein in said α-TCP powder the Si/PO₄ ratio is comprised between 0.1 and 20% in weight with respect to the total.

12. Process according to anyone of claims 7-11, comprising a further step e^{III}) wherein a powder of hydroxyapatite di-substituted with magnesium (Mg²⁺) and carbonate (CO₃)²⁻ ions is added.

13. Process according to claim 12, wherein the Mg/Ca ratio is comprised between 0.1 and 10%, preferably not less than 3%, and wherein the CO₃/PO₄ ratio is comprised between 0.1 and 20%, preferably not less than 8%.

14. Process according to anyone of claims 7-13, comprising a further step e^{V}) wherein a powder of hydroxyapatite di-substituted with strontium ions (Sr²⁺) and carbonate ions (CO₃)²⁻ is added.

15. Process according to claim 14, wherein the Sr/(Ca+Sr) ratio is comprised between 0.1 and 50%, preferably not less than 8%, and wherein the CO₃/PO₄ ratio is comprised between 0.1 and 20%, preferably not less than 8%.

16. Process according to anyone of claims 7-15, comprising a further step e^{VI}) wherein superparamagnetic hydroxyapatite substituted with Fe²⁺ and Fe³⁺ ions is added.

17. Process according to anyone of claims 7-16, wherein in step c) pellets are heated at a temperature above 1200°C, preferably at 1400°C, for a period of time comprised between 1 and 20 hours, preferably for 1 hour.

18. Process according to anyone of claims 7-17, wherein in step d) cooling is preferably carried out quickly in order to partially stabilize α-TCP against the recrystallization of the β-TCP polymorph.

19. Process according to claim 18, wherein said cooling is carried out for a period of time comprised between 0.1 and 1 minute and it is carried out until a temperature lower than the temperature needed to prevent crystallization of polymorph β-TCP is reached.

20. Process according to anyone of claims 7-19, wherein in step f) the aqueous solution comprises phosphate ions in a concentration comprised between 2.5 and 7%, preferably 5%.

21. Process according to anyone of claims 7-20, wherein the liquid/powder ratio is adjusted in the interval 0.3-0.6, preferably 0.43.

22. Injectable cement obtained with the process of anyone of claims 7-21.

23. Cement of anyone of claims 1-6 or 22 for use in bone regeneration.

24. Cement of anyone of claims 1-6 or 22 for use in vertebroplasty and kyphoplasty and in pathologies **characterized by** loss of bone substance.

25. System of drug release comprising the cement of anyone of claims 1-6 or 22.

## Patentansprüche

1. Injizierbarer Apatit-Zement, wobei Calcium im Tricalciumphosphat in der α-Phase teilweise mit Strontium substituiert ist und das Phosphat in der α-Phase wahlweise teilweise mit Silizium substituiert ist, wobei der Zement zumindest ein Polymer natürlichen Ursprungs und/oder zumindest ein bioerodierbares Material und/oder Proteine und/oder pflanzliche Polysaccharide, und wahlweise Additive zur Regulierung der Härtungszeit umfasst, und wobei Tricalciumphosphat in der β-Phase ebenfalls vorhanden ist, wobei die β-Phase des Tricalciumphosphats in einem Bereich von 20 % bis 30 % in Bezug auf die Gesamtmenge der α + β-Phasen gehalten wird.

2. Zement nach Anspruch 1, wobei im Tricalciumphosphat in der β-Phase Calcium teilweise mit Strontium und/oder Magnesium substituiert ist und Phosphat wahlweise teilweise mit Silizium substituiert ist.

3. Zement nach einem der Ansprüche 1 bis 2, wobei die Substituenten-Ionen vorzugsweise in den folgenden Verhältnissen in Bezug auf die ursprünglichen Calcium- oder Phosphat-Ionen vorliegen: 0,1 bis 50 % Strontium zu Calcium, 0 bis 20 % Silizium zu Phosphat, 0 bis 10% Magnesium zu Calcium, 0 bis 15 % Carbonat zu Phosphat, 0 bis 30 % Eisen zu Calcium.

4. Zement nach einem der Ansprüche 1 bis 3, wobei das Polymer natürlichen Ursprungs und/oder das zumindest eine bioerodierbare Polymer in einer Konzentration zwischen 0,1 und 6 Gew.-%, vorzugsweise 2 %, vorliegen, und/oder die Proteine und/oder die pflanzlichen Polysaccharide in einer Konzentration von 0 bis 50 Gew.-% vorliegen.

5. Zement nach einem der Ansprüche 1 bis 4, wobei das Polymer natürlichen Ursprungs aus der Gruppe bestehend aus Alginat, Gellan, Cellulose, Kollagen, Gelatine, Chitosan, Xanthan und Soja ausgewählt ist, und das bioerodierbare Polymer aus der Gruppe bestehend aus Polycaprolacton, Polymilchsäure, Polyacrylsäure und Polyvinylalkohol ausgewählt ist.

6. Zement nach einem der Ansprüche 1 bis 5, ferner umfassend eisensubstituiertes superparamagnetisches Hydroxylapatit.

7. Verfahren zur Herstellung des Zements nach Anspruch 1-6 in fester Phase, das folgende Schritte umfasst:
a) Zugeben von Strontiumcarbonatpulver und wahlweise Kieselsäure zu pulverförmigem Calciumcarbonat und wasserfreiem zweibasischem Dicalciumphosphat, und Trockenmischen;
b) Pressen der erhaltenen Pulver zum Herstellen von Pellets;
c) Erhitzen der Pellets auf Temperaturen über 1200 °C im Fall von Strontiumcarbonat, oder über 1000 °C im Fall von Siliziumdioxid;
d) Abkühlen mit einer Abkühlgeschwindigkeit, sodass die β-Phase von Tricalciumphosphat in Bezug auf die Gesamtmenge der α + β-Phasen im Bereich von 20 % bis 30 % bleibt;
e) Mahlen der Pellets zum Bilden eines Pulvers;
f) wahlweises Zugeben von Glycerin in einem Verhältnis von Pulver zu Glycerin in einem Bereich von 0,1 % bis 10 w/w
g) Zugeben einer wässrigen Lösung aus Natriumhydrogenphosphatdihydrat, die zumindest ein Polymer natürlichen Ursprungs und/oder zumindest ein bioerodierbares Polymer und/oder Proteine und/oder pflanzliche Polysaccharide in einer Konzentration zwischen 0,1 und 50 Gew.-% relativ zur Lösung enthält, und wahlweise Additive zur Regulierung der Härtungszeiten.

8. Verfahren nach Anspruch 7, wobei im Schritt a) Strontium in einem Sr/(Ca + Sr)-Verhältnis zwischen 0,1 und 50 % Mol vorliegt.

9. Verfahren nach einem der Ansprüche 7 bis 8, wobei im Schritt a) Silizium in einem Si/PO₄-Verhältnis zwischen 0,1 und 20 % zugegeben wird.

10. Verfahren nach einem der Ansprüche 7 bis 9, das einen weiteren Schritt e') umfasst, wobei ein mit Silizium dotiertes α-TCP-Pulver zugegeben wird.

11. Verfahren nach Anspruch 10, wobei im α-TCP-Pulver das Si/PO₄-Verhältnis zwischen 0,1 und 20 Gew.-% bezogen auf die Gesamtmenge liegt

12. Verfahren nach einem der Ansprüche 7 bis 11, das einen weiteren Schritt e^{III}) umfasst, wobei ein Pulver aus Hydroxylapatit, di-substituiert mit Magnesium (Mg²⁺)-und Carbonat (CO₃)²⁻-Ionen, zugegeben wird.

13. Verfahren nach Anspruch 12, wobei das Mg/Ca-Verhältnis zwischen 0,1 und 10 %, vorzugsweise nicht unter 3 %, liegt, und wobei das CO₃/PO₄-Verhältnis zwischen 0,1 und 20 %, vorzugsweise nicht unter 8 %, liegt.

14. Verfahren nach einem der Ansprüche 7 bis 13, das einen weiteren Schritt e^{V}) umfasst, wobei ein Pulver aus Hydroxylapatit, di-substituiert mit Strontium-Ionen (Sr²⁺) und Carbonat-Ionen (CO₃)²⁻ zugegeben wird.

15. Verfahren nach Anspruch 14, wobei das Sr/(Ca + Sr)-Verhältnis zwischen 0,1 und 50 %, vorzugsweise nicht unter 8 %, liegt, und wobei das CO₃/PO₄-Verhältnis zwischen 0,1 und 20 %, vorzugsweise nicht unter 8 %, liegt.

16. Verfahren nach einem der Ansprüche 7 bis 15, das einen weiteren Schritt e^{VI}) umfasst, wobei mit Fe²⁺- und Fe³⁺-Ionen substituierter superparamagnetischer Hydroxylapatit zugegeben wird.

17. Verfahren nach einem der Ansprüche 7 bis 16, wobei im Schritt c) Pellets für einen Zeitraum zwischen 1 und 20 Stunden, vorzugsweise 1 Stunde, auf eine Temperatur über 1200 °C, vorzugsweise auf 1400 °C, erhitzt werden.

18. Verfahren nach einem der Ansprüche 7 bis 17, wobei in Schritt d) das Abkühlen vorzugsweise schnell durchgeführt wird, um α-TCP teilweise gegen die Rekristallisation des β-TCP-Polymorphs zu stabilisieren.

19. Verfahren nach Anspruch 18, wobei das Abkühlen für einen Zeitraum von 0,1 bis 1 Minute durchgeführt wird, und durchgeführt wird, bis eine niedrigere Temperatur als die Temperatur erreicht ist, die zum Verhindern der Kristallisation von polymorphem β-TCP erforderlich ist.

20. Verfahren nach einem der Ansprüche 7 bis 19, wobei im Schritt f) die wässrige Lösung Phosphat-Ionen in einer Konzentration zwischen 2,5 und 7 %, vorzugsweise 5 %, aufweist.

21. Verfahren nach einem der Ansprüche 7 bis 20, wobei das Flüssigkeits-/Pulver-Verhältnis in den Bereich von 0,3 bis 0,6, vorzugsweise 0,43, eingestellt wird.

22. Injizierbarer Zement, der nach dem Verfahren nach einem der Ansprüche 7 bis 21 erhalten wird.

23. Zement nach einem der Ansprüche 1 bis 6 oder 22 zur Verwendung bei der Knochenregeneration.

24. Zement nach einem der Ansprüche 1 bis 6 oder 22 zur Verwendung bei der Vertebroplastie und Kyphoplastie sowie bei Pathologien, die durch Verlust der Knochensubstanz gekennzeichnet sind.

25. System zur Wirkstofffreisetzung, das den Zement nach einem der Ansprüche 1 bis 6 oder 22 umfasst.

## Revendications

1. Ciment d'apatite injectable, dans lequel le calcium dans le phosphate tricalcique en phase α est partiellement substitué par du strontium et le phosphate en phase α est optionellement partiellement substitué par du silicium, ledit ciment comprenant au moins un polymère d'origine naturelle et/ou au moins un polymère bioérodable et/ou des protéines et/ou des polysaccharides végétaux, et optionellement des additifs pour réguler le temps de durcissement, et dans lequel du phosphate tricalcique en phase β est également présent, ladite phase β du phosphate tricalcique est maintenue dans la plage de 20 % à 30 % par rapport au total des phases α + β.

2. Ciment selon la revendication 1, dans lequel dans ledit phosphate tricalcique en phase β, le calcium est partiellement substitué par du strontium et/ou du magnésium et le phosphate optionellement partiellement substitué par du silicium.

3. Ciment selon l'une quelconque des revendications 1 et 2, dans lequel les ions substituants sont de préférence présents en les rapports suivants par rapport aux ions calcium ou phosphates initiaux : de 0,1 à 50 % de strontium à calcium, de 0 à 20 % de silicium à phosphate, de 0 à 10 % de magnésium à calcium, de 0 à 15 % de carbonate à phosphate, de 0 à 30 % de fer à calcium.

4. Ciment selon l'une quelconque des revendications 1 à 3, dans lequel ledit polymère d'origine naturelle et/ou au moins un polymère bioérodable sont présents en une concentration comprise entre 0,1 et 6 % en poids, de préférence de 2 % en poids, et/ou lesdites protéines et/ou lesdits polysaccharides végétaux sont présents en une concentration de 0 à 50 % en poids.

5. Ciment selon l'une quelconque des revendications 1 à 4, dans lequel ledit polymère d'origine naturelle est sélectionné dans le groupe consistant en l'alginate, le gellane, la cellulose, le collagène, la gélatine, le chitosane, le xanthane et le soja et ledit polymère bioérodable est sélectionné dans le groupe consistant en la polycaprolactone, l'acide polylactique, l'acide polyacrylique et l'alcool polyvinylique.

6. Ciment selon l'une quelconque des revendications 1 à 5, comprenant en outre de l'hydroxyapatite superparamagnétique substituée par du fer.

7. Procédé de préparation en phase solide du ciment selon les revendications 1 à 6 comprenant les étapes suivantes :
a) ajout de poudre de carbonate de strontium et optionellement de silice au carbonate de calcium et au phosphate dicalcique dibasique anhydre pulvérulents et mélange à sec ;
b) compression des poudres obtenues pour produire des granules ;
c) chauffage desdits granules à des températures supérieures à 1 200 °C dans le cas du carbonate de strontium ou supérieures à 1 000 °C dans le cas de la silice ;
d) refroidissement à une vitesse de refroidissement telle que la phase β du phosphate tricalcique reste dans la plage de 20 % à 30 % par rapport au total des phases α + β ;
e) broyage desdits granules pour former une poudre ;
f) optionellement ajout de glycérol dans une poudre à un rapport de glycérol se trouvant dans la plage de 0,1 % à 10 % p/p ;
g) ajout d'une solution aqueuse d'hydrogénophosphate de sodium dihydrate contenant au moins un polymère d'origine naturelle et/ou au moins un polymère bioérodable et/ou des protéines et/ou des polysaccharides végétaux en une concentration comprise entre 0,1 et 50 % en poids par rapport à ladite solution, et optionellement des additifs pour réguler les temps de durcissement.

8. Procédé selon la revendication 7, dans lequel à ladite étape a) le strontium est présent en un rapport Sr/(Ca+Sr) compris entre 0,1 et 50 % en moles.

9. Procédé selon l'une quelconque des revendications 7 et 8, dans lequel le silicium est ajouté à l'étape a) en un rapport Si/PO₄ compris entre 0,1 et 20 %.

10. Procédé selon l'une quelconque des revendications 7 à 9, comprenant une étape e^{I}) supplémentaire dans laquelle une poudre d'α-TCP dopé au silicium est ajoutée.

11. Procédé selon la revendication 10, dans lequel dans ladite poudre d'α-TCP le rapport Si/PO₄ est compris entre 0,1 et 20 % en poids par rapport au total.

12. Procédé selon l'une quelconque des revendications 7 à 11, comprenant une étape e^{III}) supplémentaire dans laquelle une poudre d'hydroxyapatite disubstituée avec des ions magnésium (Mg²⁺) et carbonate (CO₃)²⁻ est ajoutée.

13. Procédé selon la revendication 12, dans lequel le rapport Mg/Ca est compris entre 0,1 et 10 %, de préférence pas inférieur à 3 %, et dans lequel le rapport CO₃/PO₄ est compris entre 0,1 et 20 %, de préférence pas inférieur à 8 %.

14. Procédé selon l'une quelconque des revendications 7 à 13, comprenant une étape e^{V}) supplémentaire dans laquelle une poudre d'hydroxyapatite disubstituée par des ions strontium (Sr²⁺) et des ions carbonate (CO₃)²⁻ est ajoutée.

15. Procédé selon la revendication 14, dans lequel le rapport Sr/(Ca+Sr) est compris entre 0,1 et 50 %, de préférence pas inférieur à 8 %, et dans lequel le rapport CO₃/PO₄ est compris entre 0,1 et 20 %, de préférence pas inférieur à 8 %.

16. Procédé selon l'une quelconque des revendications 7 à 15, comprenant une étape e^{VI}) supplémentaire dans laquelle de l'hydroxyapatite superparamagnétique substituée par des ions Fe²⁺ et Fe³⁺ est ajoutée.

17. Procédé selon l'une quelconque des revendications 7 à 16, dans lequel à l'étape c) les granules sont chauffés à une température supérieure à 1 200 °C, de préférence à 1 400 °C, pendant une période de temps comprise entre 1 et 20 heures, de préférence de 1 heure.

18. Procédé selon l'une quelconque des revendications 7 à 17, dans lequel à l'étape d) le refroidissement est de préférence réalisé rapidement de manière à partiellement stabiliser l'a-TCP contre la recristallisation du polymorphe de β-TCP.

19. Procédé selon la revendication 18, dans lequel ledit refroidissement est réalisé pendant une période de temps comprise entre 0,1 et 1 minute et est réalisé jusqu'à ce qu'une température inférieure à la température nécessaire pour empêcher la cristallisation du polymorphe β-TCP soit atteinte.

20. Procédé selon l'une quelconque des revendications 7 à 19, dans lequel à l'étape f) la solution aqueuse comprend des ions phosphate en une concentration comprise entre 2,5 et 7 %, de préférence de 5 %.

21. Procédé selon l'une quelconque des revendications 7 à 20, dans lequel le rapport liquide/poudre est ajusté dans l'intervalle de 0,3 à 0,6, de préférence à 0,43.

22. Ciment injectable obtenu avec le procédé selon l'une quelconque des revendications 7 à 21.

23. Ciment selon l'une quelconque des revendications 1 à 6 ou 22 pour son utilisation dans la régénération osseuse.

24. Ciment selon l'une quelconque des revendications 1 à 6 ou 22 pour son utilisation en vertébroplastie et en cyphoplastie et dans des pathologies **caractérisées par** la perte de substance osseuse.

25. Système de libération de médicament comprenant le ciment selon l'une quelconque des revendications 1 à 6 ou 22.
